# EUROPEAN PATENT SPECIFICATION

(11) **EP 2 909 161 B1**
(45) Date of publication and mention of the grant of the patent: **06.09.2017**
(21) Application number: 13779714.8
(22) Date of filing: 10.10.2013
(51) Int. Cl.: C07C 17/25, C07C 17/275, C08F 214/22, C07C 19/16, C07C 21/17, C07C 21/18

(54) **FLUOROIODO COMPOUNDS FOR FLUOROPOLYMERS**
FLUORIODVERBINDUNGEN FÜR FLUORPOLYMERE
COMPOSÉS FLUORO-IODO POUR FLUOROPOLYMÈRES

(30) Priority: 18.10.2012 US 201261715413 P
(43) Date of publication of application: 26.08.2015
(73) Proprietor: 3M Innovative Properties Company, St. Paul, MN 55133-3427 (US)
(72) Inventor: GUERRA, Miguel A., Saint Paul, Minnesota 55133-3427 (US); FUKUSHI, Tatsuo, Saint Paul, Minnesota 55133-3427 (US); QIU, Zai-Ming, Saint Paul, Minnesota 55133-3427 (US); CLEM, Tabitha A., Saint Paul, Minnesota 55133-3427 (US)
(74) Representative: Herzog, Fiesser & Partner Patentanwälte PartG mbB
(86) International application number: PCT/US2013/064249
(87) International publication number: WO 2014/062469

(56) References cited:
- EP-A2- 1 262 497
- WO-A1-2012/082707
- WO-A2-2012/082703
- WO-A2-2012/083107
- MANSERI A ET AL: "Synthesis of telechelic dienes from fluorinated alpha,omega-diiodoalkanes. Part I. Divinyl and diallyl derivatives from model I(C2F4)nI compounds", JOURNAL OF FLUORINE CHEMISTRY, ELSEVIER, NL, vol. 73, no. 2, 1 August 1995 (1995-08-01) , pages 151-158, XP004020530, ISSN: 0022-1139, DOI: 10.1016/J.JFLUCHEM.2005.06.007
- SLOAN JOHN P ET AL: "Free radical addition to olefins. XVI. Photolysis of difluoroiodomethane in the presence of olefins", JOURNAL OF THE CHEMICAL SOCIETY, PERKIN TRANSACTIONS 2: PHYSICAL ORGANIC CHEMISTRY, ROYAL SOCIETY OF CHEMISTRY, GB, no. 15, 1 January 1972 (1972-01-01), pages 1841-1845, XP009176873, ISSN: 0300-9580

## Description

### TECHNICAL FIELD

Fluoroiodo compounds are described, along with methods of making thereof. These fluoroiodo compounds may be used in the preparation of fluoropolymers.

### SUMMARY

There is a desire to find alternative fluoroiodo compounds for use in polymer synthesis. There is also a desire to prepare these fluoroiodo compounds having an odd number of carbons. It would also be desirable to identify methods of making these fluoroiodo compounds that may be more efficient and/or cheaper than traditional methods.

In one aspect, a method of making a partially fluorinated compound is provided comprising: (a) reducing a perfluorodisulfonyl fluoride to form a disulfinate; (b) reacting the disulfinate with iodine to form a perfluorinated diiodo-compound; and (c) reacting the perfluorinated diiodido-compound with ethylene to form an ethylene substituted perfluorodiiodide. In one embodiment, the ethylene substituted perfluorodiiodide is reacted with a base to form a partially fluorinated iodo alkene compound.

In one aspect, a composition is provided comprising a partially fluorinated iodo compound selected from the group consisting of: I(CF₂)ₓCH=CH₂ wherein x is an odd integer selected from 3 to 11.

In another aspect, a polymer composition is provided comprising the polymerized reaction product of: a composition comprising a partially fluorinated iodo compound selected from the group consisting of: I(CF₂)ₓCH=CH₂ wherein x is an odd integer selected from 3 to 11; and a fluorinated olefinic monomer.

In another aspect, a method of making a polymer is described comprising: (a) providing the partially fluorinated compound, I(CF₂)ₓCH=CH₂ wherein x is an odd integer selected from 3 to 11; a fluorinated olefinic monomer; and an initiator; and (b) polymerizing the partially fluorinated compound and the fluorinated olefinic monomer to form a polymer.

The above summary is not intended to describe each embodiment. The details of one or more embodiments of the invention are also set forth in the description below. Other features, objects, and advantages will be apparent from the description and from the claims.

### DETAILED DESCRIPTION

As used herein, the term
"a", "an", and "the" are used interchangeably and mean one or more; and
"and/or" is used to indicate one or both stated cases may occur, for example A and/or B includes, (A and B) and (A or B).

Also herein, recitation of ranges by endpoints includes all numbers subsumed within that range (e.g., 1 to 10 includes 1.4, 1.9, 2.33, 5.75, 9.98, etc.).

Also herein, recitation of "at least one" includes all numbers of one and greater (e.g., at least 2, at least 3, at least 4, at least 6, at least 8, at least 10, at least 25, at least 50, at least 100, etc.).

α,ω-Diiodoperfluoroalkanes are important building blocks in the preparation of other fluorinated compounds and polymers. In fluoropolymers, the α,ω-diiodoperfluoroalkanes are used as chain transfer agents, helping to control the molecular weight of the polymer. Typically, these α,ω-diiodoperfluoroalkanes are made from oligomerization of tetrafluoroethylene and iodine resulting in even numbered CF₂ units. See, for example J. Org. Chem., v.42, no. 11, p. 1985-1990 (1977). Further, iodo-compounds can be polymerized into the polymer and the presence of iodide in fluoropolymer is useful for crosslinking.

Fluorinated diiodides with an odd number of CF₂ units have been difficult and costly prepare. Fluorinated diiodides with an odd number of CF₂ units have been made by reacting ICF₂I with tetrafluoroethylene, however, tetrafluoroethylene can be hard to handle and ICF₂I is not readily available.

The present disclosure is directed to a one pot, two-step synthesis for preparing α,ω-diiodoperfluoroalkanes (i.e., I(CF₂)ₙI, where n is at least 1). This method can be used to prepare α,ω-diiodoperfluoroalkanes having high yields. Advantageously, this method may be used to prepare α,ω-diiodoperfluoroalkanes having an odd number of CF₂ units. Also described herein is a method for preparing partially fluorinated α,ω-diiodoalkanes and partially fluorinated iodoolefins from α,ω-diiodoperfluoroalkanes.

### Method I

Disclosed herein, is an efficient (one pot, two-step) synthesis for preparing perfluorinated diiodo-compounds, wherein perfluorodisulfonyl fluoride is reduced to form a disulfinate, which is then reacted with iodine to form a perfluorinated diiodo-compound. In one embodiment, the perfluorinated diiodo-compound is an α,ω-diiodoperfluoroalkane (i.e., I(CF₂)ₙI, where n is at least 1, e.g., n is 1, 3, 5, 7, 9 or 11).

The first step of the synthesis in Method I involves reducing a perfluorodisulfonyl fluoride.

The perfluorodisulfonyl fluoride of the present disclosure is a compound of formula FSO₂-R_{f}-SO₂F, wherein R_{f} is a divalent, perfluorinated linking group. The perfluorodisulfonyl fluoride may be made using techniques known in the art, including for example, electrochemical fluorination. Typically the perfluorinated linking group comprises at least 3, 5, 7 or even 9 carbon atoms. The perfluorinated linking group is linear. The perfluorinated linking group is a perfluorinated hydrocarbon.

Exemplary perfluorodisulfonyl fluoride compounds include: FSO₂-CF₂CF₂CF₂-SO₂F, and FSO₂-CF₂ CF₂CF₂CF₂CF₂-SO₂F.

Reducing agents as known in the art can be used to reduce the perfluorodisulfonyl fluoride. Exemplary reducing agents useful in the present disclosure include those represented by the formula, M'Y'H₄, wherein M' is an alkali metal or an alkaline Earth metal and Y' is Aluminum or Boron, including, for example, sodium borohydride, sodium cyanoborohydride, potassium borohydride, lithium borohydride, and lithium aluminum hydride. Useful hydride reducing agents also include those represented by the formula, M"Hₙ, wherein M" is an alkali metal, and n is an integer selected from 1 or 2, including, for example, sodium hydride, lithium hydride, potassium hydride, barium hydride, and calcium hydride. Other useful reducing agents include mono-, di-, or tri(lower alkoxy) alkali metal aluminum hydrides, mono-, di-, or tri-(lower alkoxy lower alkoxy) alkali metal aluminum hydrides, di(lower alkyl) aluminum hydrides, alkalimetalcyanoborohydrides, tri(loweralkyl) tin hydrides, tri(aryl) tin hydrides, Li(C₂H₅)₃BH, and (((CH₃)₂CHCH₂)₂AlH)₂. Another useful reducing agent for the perfluorodisulfonyl fluoride is an alkaline sulfite. Useful alkaline sulfites include, alkali metal and alkaline earth metal sulfites, for example, K₂SO₃, Na₂SO₃, KHSO₃, and NaHSO₃.

The reduction of the perfluorodisulfonyl fluoride may be done in the presence of a solvent. The selection of the solvent may depend on the reducing agent used. The solvent should be inert to the reactants and product and the reactants and the product should have at least some solubility in the solvent. Exemplary solvents include polar aprotic solvents such as, CH₃CN, dimethylformamide, dimethyl sulfoxide, and other solvents such as dialkyl ethers (e.g., diethyl ether, t-butyl methyl ether, glycol dialkyl ether (e.g., CH₃OCH₂CH₂OCH₃), dioxane, and tetrahydrofuran), and combinations thereof. Exemplary solvents also include polar protic solvents such as lower alkanols, having between 1 and 4 carbon atoms (e.g., methanol, ethanol, isopropanol, n-butanol, etc.), acids (e.g. acetic acid), water, and combinations thereof.

The reduction of the perfluorodisulfonyl fluoride may be conducted at a temperature of at least 10, 20, 23, 25, 30, or even 35°C; at most 70, 80, 90, 100, 150, 200, or even 220°C.

The reduced perfluorosulfonyl fluoride then is reacted with iodine to form the perfluorinated diiodo compound (e.g., an α,ω-diiodoperfluoroalkanes).

In one embodiment, before contacting with the iodine, the reaction product from the reducing step above, first may be acidified to make the disulfinic acid. Acidifying the reaction product can allow for removal of insoluble salts before contacting with the iodine. If acidifying, acids as known in the art, including hydrochloric acid, sulfuric acid, and phosphoric acid may be used to achieve the desired acidity.

To form the desired perfluorinated diiodo-compound, the disulfinate reaction product from above is reacted with iodine.

In order to get the substitution reaction to occur, either a radical forming compound (a radical initiator or electron-donor) and/or heat is used. For example, persulfate can be used to disassociate the sulfinic group forming a radical, which can then react with iodine to form the perfluorinated diiodo-compound. In another example, the reaction mixture comprising the disulfinate can be heated above the decomposition temperature of the sulfinic group to generate a radical, which can then react with iodine to form the perfluorinated diiodo-compound.

In one embodiment, the reaction with the iodine is conducted in the presence of a solvent. Typical solvents include, for example, water, acetonitrile, isopropanol, acetic acid and combinations thereof.

In one embodiment the reaction with iodine may be conducted at a temperature of between at least 10, 20, 23, 25, 30, or even 35°C; at most 70, 80, 90, 100, 150, 200, or even 220°C.

The resulting perfluorinated diiodo compound may be isolated and purified by known methods.

The number of carbons in the resulting perfluorinated diiodo compound is identical to the number of carbons present in the perfluorodisulfonyl fluoride starting material. Therefore, Method I as disclosed above may be used to generate odd numbered α,ω-diiodoperfluoroalkanes. For example, if the perfluorodisulfonyl fluoride comprises three CF₂ groups than the resulting α,ω-diiodoperfluoroalkane will have three CF₂ groups.

Exemplary perfluorinated diiodo compounds made by the process described above include α,ω-diiodoperfluoroalkanes having the structure of: I-CF₂CF₂CF₂-I, and I-CF₂ CF₂ CF₂C_{F2}C_{F2} -I.

In one embodiment, the perfluorinated diiodo compounds of the present disclosure may be used as chain transfer agents in polymer syntheses or, as will be described below, can be used to generate other fluorinated compounds.

### Method II

Also disclosed herein is a process for making an ethylene substituted perfluorodiiodide (e.g., an α,ω-diiodohydrofluoroalkanes), wherein a perfluorinated diiodo-compound is reacted with ethylene to form an α ethylene substituted perfluorodiiodide. For example, a compound according to Formula I is reacted with ethylene to form a compound according to Formula II.

I(CF₂)ₙI (Formula I) + CH₂=CH₂ → ICH₂CH₂(CF₂)ₙI (Formula II)

wherein n is at least 1. In one embodiment of the present application, n is an odd integer of at least 3, 5, 7, or even 9. Note that although Method II illustrates linear alkane compounds, the chemistries described in Method II may be applied similarly to other perfluorinated diiodo-compounds disclosed herein.

In one embodiment, an α,ω-diiodoperfluoroalkane (e.g., the compound according to Formula I) may be made according to Method I disclosed above or can be made by reacting hexafluoropropylene oxide with iodine as disclosed in, for example, U.S. Appl. No. 61/715059 (filed 10-17-2012). Alternatively, the α,ω-diiodoperfluoroalkane can be made by a process including known processes of making α,ω-diiodoperfluoroalkanes, such as TFE telomerization. However, these alternate processes may not favor the formation of odd numbered carbon chain length, which may be desirable in some instances.

Ethylene is then added to the perfluorinated diiodo-compound (e.g., α,ω-diiodoperfluoroalkanes). To achieve the insertion of the ethylene, either a radical forming compound, light (such as UV radiation), and/or heat is used.

Exemplary radical forming compounds include, peroxides or azo compounds. Peroxides include, for example, organic peroxides, such as diacyl peroxides, peroxyesters, dialkyl peroxides, and hyrdoperoxides. Azo compounds include, for example, azoisobutyronitrile and azo-2-cyanovaleric acid. Other radical forming compounds are electron donors, such as metal or metal complexes with ligands as well known in the literature. Exemplary electron donor for such addition of perfluorinated iodide with unsaturated carbon-carbon bond are Cu, Zn, Mg, Pd(0), Fe, Ni, Pt(P(C₆H₅)₃)₄, Ir(CO)H(P(C₆H₅)₃)₃, Pb(C₂H₃O₂)₄ and RhCl(P(C₆H₅)₃)₂.

In one embodiment the reaction may be conducted at a temperature of at least 10, 25, 50, 100, or even 125°C; at most 140, 150, 200, or even 220°C.

In one embodiment, the ratio of ethylene to α,ω-diiodoperfluoroalkane is 1:2 to 2:1, more preferably 1:0.8 to 1:1.1, even more preferably, 1:0.9.

In one embodiment, the reaction with the ethylene is conducted neat. In another embodiment, the reaction with the ethylene is conducted in the presence of a solvent. Typical solvents include inert solvents, for example, fluorinated solvents such as those available under the trade designation "3M FLUORINERT ELECTRONIC LIQUID" and "3M NOVEC ENGINEERED FLUID" from 3M Co., St. Paul, MN.

The resulting ethylene substituted perfluorodiiodide (e.g., α,ω)-diiodohydrofluoroalkane) may be isolated and purified by known methods.

Exemplary ethylene substituted perfluorodiiodides include: I-CH₂CH₂CF₂CF₂CF₂-I, and I-CH₂CH₂CF₂CF₂CF₂CF₂CF₂-I.

In one embodiment, Formula II is I(CF₂)ₓCH₂CH₂I wherein x is an odd integer selected from 3 to 11 (in other words 3, 5, 7, 9, or 11).

In one embodiment, the ethylene substituted perfluorodiiodides of the present disclosure may be used as chain transfer agents in polymer syntheses or can also be used to generate other fluorinated compounds.

### Method III

Also disclosed herein is a process for making partially fluorinated terminal iodo alkenes which, in one embodiment, can be used as a cure site monomer.

In Method III of the present disclosure, a ethylene substituted perfluorodiiodide is dehydroiodinized to form a partially fluorinated iodo alkene. For example, a compound according to Formula II is dehydroiodinated to form a compound according to Formula III.

ICH₂CH₂(CF₂)ₙI (Formula II) → CH₂=CH(CF₂)ₙI (Formula III)

wherein n is an odd integer of at least 1, 3,5,7, or even 9.

In the present disclosure the ethylene substituted perfluorodiiodide from Method II may be further treated with a base or base-like compound in an alcoholic solution to form a partially fluorinated terminal iodo alkene.

Generally, at least a mole equivalent of the base or base-like compound to the ethylene substituted perfluorodiiodide should be used to favor the formation of the partially fluorinated iodo alkene compound.

Base and base-like compounds include those known in the art, for example, methoxides, KOH, NaOH, alkyl amines, LiCl in dimethylformamide, etc.

In one embodiment the reaction may be conducted at a temperature of at least 10, 20, 23, 25, 30, or even 35°C; at most 70, 80, 90, 100, 150, 200, or even 220°C.

In one embodiment, the reaction is conducted in the presence of a solvent. Typical solvents include, for example, water and polar organic solvents such as lower alkanols including ethanol, methanol, isopropanol, butanol, etc.

The resulting partially fluorinated terminal iodo alkene compounds may be isolated and purified by known methods.

Exemplary partially fluorinated iodo alkene compounds include: CH=CH₂CF₂CF₂CF₂-I, and CH₂=CH₂CF₂CF₂CF₂CF₂CF₂-I.

Formula III is I(CF₂)ₓCH₂=CH₂ wherein x is an odd integer selected from 3 to 11 (in other words 3, 5, 7, 9, or 11).

In one embodiment, the partially fluorinated terminal iodo alkenes of the present disclosure may be used as cure site monomers in polymer syntheses.

### Polymer Synthesis

In one embodiment, the compounds of Formulas I, II, and III can be used either individually or combined during fluoropolymer polymerization (e.g., as a cure site monomer or a chain transfer agent).

In preparing fluoropolymers, the compounds of Formulas I, II, and/or Formula III may be polymerized with one or more fluorinated olefinic monomer(s) to form a polymer.

A fluorinated olefinic monomer is a monomer having a carbon-carbon double bond and comprising at least one fluorine atom. The fluorinated olefinic monomer may be perfluorinated (or fully fluorinated) or partially fluorinated (comprising at least one hydrogen atom and one fluorine atom).

Exemplary perfluorinated olefinic monomers include: hexafluoropropene (HFP), tetrafluoroethylene (TFE), pentafluoropropylene, trifluorochloroethylene (CTFE), perfluoro(alkylvinyl ether), chlorotrifluoroethylene, perfluoro(methyl vinyl ether) (PMVE), perfluoro(propyl vinyl ether) (PPVE), perfluoro(methoxypropyl vinyl ether), perfluoro(ethoxymethyl vinyl ether), CF₂=CFOCFCF₂CF₂OCF₃, CF₂=CFOCF₂OCF₂CF₂CF₃, CF₂=CFOCF₂OCF₂CF₃, CF₂=CFOCF₂OCF₃, and combinations thereof.

Exemplary partially fluorinated olefinic monomers include: vinyl fluoride (VF), vinylidene fluoride (VDF), fluoroethylene, pentafluoropropylene (e.g., 2-hydropentafluropropylene), trifluoroethylene, and combinations thereof.

In addition to the fluorinated olefinic monomer, additional monomers may be added, such as non-fluorinated olefinic monomers. Exemplary non-fluorinated olefinic monomers include: propylene, ethylene, isobutylene, and combinations thereof. Generally, these additional monomers would be used at less than 25 mole percent of the fluoropolymer, preferably less than 10 mole percent, and even less than 3 mole percent.

In the present disclosure, the compound according to Formula I and/or II may be used in the polymerization as a chain transfer agent. Chain transfer agents are added to the polymerization to control the molecular weight of the growing polymer chain.

In the present disclosure, the compound according to Formula III may be used in the polymerization as a cure site monomer. Cure site monomers are polymerized into the polymer during polymerization and are then used as sites to subsequently crosslink polymer chains.

Initiators as known in the art can be used to initiate the polymerization of the fluorinated olefinic monomers with the cure-site monomers and/or chain transfer agents of the present disclosure.

When using the cure site monomer of Formula III during a polymerization, the chain transfer agent of Formula II may be used and/or a chain transfer agent selected from (i) a C1 to C10 α,ω-diiodoperfluoroalkane; (ii) I(CF₂)_{y}CH₂CH₂I, wherein y is an even integer from 2 to 10; (iii) CH₂I₂; and (iv) combinations thereof. Exemplary chain transfer agents include 1,3-diiodoperfluoropropane, 1,4- diiodoperfluorobutane, 1,6-diiodoperfluorohexane, and 1,8- diiodoperfluorooctane.

After polymerization, the polymer dispersion may be coagulated and washed as is known in the art to form a polymer gum.

In one embodiment the polymer of the present disclosure comprises at least 0.05, 0.1, 0.2 or even 0.4 % by weight iodine relative to the total weight of the polymer gum. In one embodiment the polymer gum of the present disclosure comprises at most 0.5, 0.75, 1, or even 1.5 % by weight iodine relative to the total weight of the polymer gum.

The polymer gums of the present disclosure are partially fluorinated elastomers. As disclosed herein a partially fluorinated elastomer is an amorphous polymer comprising at least one hydrogen and at least one fluorine atom on the backbone of the polymer.

Exemplary fluoropolymers include: a TFE/propylene copolymer, a TFE/propylene/VDF copolymer, a VDF/HFP copolymer, a TFE/VDF/HFP copolymer, a TFE/PMVE copolymer, a TFE/CF₂=CFOC₃F₇ copolymer, a TFE/CF₂=CFOCF₃/CF₂=CFOC₃F₇ copolymer, a TFE/CF₂=C(OC₂F₅)₂ copolymer, a TFE/ethyl vinyl ether (EVE) copolymer, a TFE/butyl vinyl ether (BVE) copolymer, a TFE/EVE/BVE copolymer, a VDF/CF₂=CFOC₃F₇ copolymer, an ethylene/HFP copolymer, a TFE/ HFP copolymer, a CTFE/ VDF copolymer, a TFE/VDF copolymer, a TFE/VDF/PMVE/ethylene copolymer, and a TFE/ VDF/ CF₂=CFO(CF₂)₃OCF₃ copolymer.

### Curing

The fluoropolymer of the present disclosure may be cured with peroxide curing agents including organic peroxides. In many cases it is preferred to use a tertiary butyl peroxide having a tertiary carbon atom attached to a peroxy oxygen.

Exemplary peroxides include: 2,5-dimethyl-2,5-di(t-butylperoxy)hexane; dicumyl peroxide; di(2-t-butylperoxyisopropyl)benzene; dialkyl peroxide; bis (dialkyl peroxide); 2,5-dimethyl-2,5-di(tertiarybutylperoxy)3-hexyne; dibenzoyl peroxide; 2,4-dichlorobenzoyl peroxide; tertiarybutyl perbenzoate; α,α'-bis(t-butylperoxy-diisopropylbenzene); t-butyl peroxy isopropylcarbonate, t-butyl peroxy 2-ethylhexyl carbonate, t-amyl peroxy 2-ethylhexyl carbonate, t-hexylperoxy isopropyl carbonate, di[1,3-dimethyl-3-(t-butylperoxy)butyl] carbonate, carbonoperoxoic acid, O,O'-1,3-propanediyl OO,OO'-bis(1,1-dimethylethyl) ester, and combinations thereof.

The amount of peroxide curing agent used generally will be at least 0.1, 0.2, 0.4, 0.6, 0.8, 1, 1.2, or even 1.5; at most 2, 2.25, 2.5, 2.75, 3, 3.5, 4, 4.5, 5, or even 5.5 parts by weight per 100 parts of fluoropolymer.

In peroxide cure systems, it is often desirable to include a coagent. Those skilled in the art are capable of selecting conventional coagents based on desired physical properties. Exemplary coagents include: tri(methyl)allyl isocyanurate (TMAIC), triallyl isocyanurate (TAIC), tri(methyl)allyl cyanurate, poly-triallyl isocyanurate (poly-TAIC), triallyl cyanurate (TAC), xylylene-bis(diallyl isocyanurate) (XBD), N,N'-m-phenylene bismaleimide, diallyl phthalate, tris(diallylamine)-s-triazine, triallyl phosphite, 1,2-polybutadiene, ethyleneglycol diacrylate, diethyleneglycol diacrylate, and combinations thereof. Another useful coagent may be represented by the formula CH₂=CH-R_{f1}-CH=CH₂ wherein R_{f1} may be a perfluoroalkylene of 1 to 8 carbon atoms. Such coagents provide enhanced mechanical strength to the final cured elastomer. They generally are used in amount of at least 0.5, 1, 1.5, 2, 2.5, 3, 4, 4.5, 5, 5.5, or even 6; at most 4, 4.5, 5, 5.5, 6, 7, 8, 9, 10, 10.5, or even 11 parts by weight per 100 parts of the fluoropolymer.

The fluoropolymer compositions can also contain a wide variety of additives of the type normally used in the preparation of elastomeric compositions, such as pigments, fillers (such as carbon black), pore-forming agents, and those known in the art.

Metal oxides are traditionally used in peroxide curing. Exemplary metal oxides include: Ca(OH)₂, CaO, MgO, ZnO, and PbO. In one embodiment, the curable fluoropolymer is essentially free of metal oxide (i.e., the composition comprises less than 1, 0.5, 0.25, 0.1, or even less than 0.05 parts per 100 parts of the fluoroelastomer). In one embodiment, the curable fluoropolymer comprises metal oxide. For example, at least 1.5, 2, 4, 5, or even 6 parts metal oxide per 100 parts of the fluoropolymer.

In the present curing process, the fluoropolymer gum, along with the required amounts of peroxide, coagent, and other components, is compounded by conventional means, such as in a two-roll mill, at elevated temperatures. The fluoropolymer gum is then processed and shaped (for example, in the shape of a hose or hose lining) or molded (for example, in the form of an O-ring). The shaped article can then be heated to cure the gum composition and form a cured elastomeric article.

The cured fluoroelastomers are particularly useful as seals, gaskets, and molded parts in systems that are exposed to elevated temperatures and/or corrosive materials, such as in automotive, chemical processing, semiconductor, aerospace, and petroleum industry applications, among others. Because the fluoroelastomers may be used in sealing applications, it is important that the elastomers perform well under compression. Compressive sealing is based on the ability of an elastomer to be easily compressed and develop a resultant force that pushes back on the mating surfaces. The ability of a material to maintain this resultant force as a function of time over a range of environmental conditions is important to long term stability. As a result of thermal expansion, stress relaxation, and thermal aging, the initial sealing forces will decay over time. By determining the retained sealing force, elastomeric materials can be evaluated for their sealing force retention under a range of conditions, particularly under high temperature conditions, such as 200°C, 225°C, 250°C, and even 275°C.

Exemplary embodiments of the present disclosure include:
Item 1. A composition comprising a partially fluorinated compound of: I(CF₂)ₓCH=CH₂ (Formula III); wherein x is an odd integer selected from 3 to 11.
Item 2. A polymer composition comprising the polymerized reaction product of: the partially fluorinated compound of item 1; and a fluorinated olefinic monomer.
Item 3. The polymer composition of item 2, wherein the fluorinated olefinic monomer is selected from the group consisting of: hexafluoropropylene, trifluoroethylene, fluoroethylene, vinylidene fluoride, tetrafluoroethylene, perfluoro(methyl vinyl ether), perfluoro(propyl vinyl ether), perfluoro(methoxypropyl vinyl ether), perfluoro(ethoxymethyl vinyl ether), chlorotrifluoroethylene, and combinations thereof.
Item 4. The polymer composition of any one of items 2 or 3, wherein the polymerized reaction product further comprises a chain transfer agent, wherein the chain transfer agent is selected from the group consisting of: a C1 to C10 α,ω-diiodoperfluoroalkane; I(CF₂)_{y}CH₂CH₂I, wherein y is an integer from 2 to 10; CH₂I₂; and combinations thereof.
Item 5. The polymer composition of item 4, wherein the chain transfer agent is 1,3-diiodoperfluoropropane or 1,4- diiodoperfluorobutane.
Item 6. The polymer composition of any one of items 2 to 5, wherein x is 3.
Item 7. The polymer composition of any one of items 2 to 6, wherein the polymerized reaction product further comprises a non-fluorinated olefinic monomer.
Item 8. The polymer composition of any one of items 2 to 7, wherein the polymer composition comprises 0.05 to 1 % by weight of iodine.
Item 9. An article comprising the cured polymer composition according to any one of items 2 to 8.
Item 10. A method of making a polymer comprising: providing the partially fluorinated compound, I(CF₂)ₓCH=CH₂ (Formula III) of item 1; a fluorinated olefinic monomer; and an initiator; and polymerizing the partially fluorinated compound and the fluorinated olefinic monomer to form a polymer.
Item 11. The method of item 10, further comprising polymerizing in the presence of a chain transfer agent.
Item 12. The method of item 11, wherein the chain transfer agent is 1,3-diiodoperfluoropropane or 1,4- diiodoperfluorobutane.
Item 13. The method of any one of items 10-12, wherein the fluorinated olefinic monomer is selected from the group consisting of: hexafluoropropylene, trifluoroethylene, fluoroethylene, vinylidene fluoride, tetrafluoroethylene, perfluoro(methyl vinyl ether), perfluoro(propyl vinyl ether), perfluoro(methoxypropyl vinyl ether), perfluoro(ethoxymethyl vinyl ether), chlorotrifluoroethylene, and combinations thereof.
Item 14. A method of making a perfluorinated compound comprising: reducing a perfluorodisulfonyl fluoride to form a disulfinate; and reacting the disulfinate with iodine to form a α,ω-diiodoperfluoroalkane.
Item 15. A method of making a perfluorinated compound comprising the following steps in order: providing hexafluoropropylene oxide and iodine; and reacting the hexafluoropropylene oxide with the iodine to form a α,ω-diiodoperfluoroalkane of the formula I(CF₂)ₓI, wherein x is an odd integer selected from 3 to 11
Item 16. A method of making a partially fluorinated compound comprising reacting an α,ω-diiodoperfluoroalkane from Items 14 or 15 with ethylene to form an α,ω-diiodohydrofluoroalkane.
Item 17. The method of item 16, further comprising dehydroiodinating the α,ω-diiodohydrofluoroalkane to form an iodinated partially fluorinated olefin of the formula I(CF₂)ₓCH=CH₂, wherein x is at least 1.

### EXAMPLES

Advantages and embodiments of this disclosure are further illustrated by the following examples, but the particular materials and amounts thereof recited in these examples, as well as other conditions and details, should not be construed to unduly limit this invention. In these examples, all percentages, proportions and ratios are by weight unless otherwise indicated.

All materials are commercially available, for example from Sigma-Aldrich Chemical Company; Milwaukee, WI, or known to those skilled in the art unless otherwise stated or apparent.

These abbreviations are used in the following examples: g = gram, min = minute, mol=mole, hr = hour, mL = milliliter; wt = weight.

**Materials**

| Material Name | Description |
|---|---|
| Iodine | Available from Alpha Aesar, A Johnson Matthey Company, Ward Hill, MA |
| Nickel catalyst | Available under the trade designation "PRO-PAK DISTILLATION PACKING (0.24") " from Cannon Instrument Company, State College, Pennsylvania. This is a nickel ribbon with over 1000 tiny holes per in² (over 155 tiny holes per cm²). |
| HFPO | Hexafluoropropylene oxide, available from E.I. DuPont de Nemours and Company, Wilmington, DE |
| Perfluoropropane disulfonyl fluoride | FSO₂C₃F₆SO₂F can be made as described by E. Hollitzer and P. Sartori, "The Electrochemical Perfluoroination (ECPF) of Propanesulfonyl Fluorides. Part I. Preparation and ECPF of 1-Propanesulfonyl Fluoride and 1,3-Propanedisulfonyl Difluoride" Journal of Fluorine Chemistry, 35 (1987) 329-341. |
| Sodium borohydride | Available from Sigma-Aldrich Chemical Company |
| Sodium persulfate | Available from Sigma-Aldrich Chemical Company |
| t-Amyl-2-ethyl hexanoate peroxide | Available from United Initiators, Inc., Elyria, OH |
| 1,4-Diiodooctafluorobutane | I(CF₂)₄I, available from FSUE Russian Scientific Center of Applied Chemistry Perm Branch, Russia. |
| N990 carbon black | Available under the trade designation "THERMAX FLOFORM MEDIUM THERMAL CARBON BLACK N990", ASTM N990 from Cancarb Ltd., Medicine Hat, Alberta, Canada |
| Zinc oxide | Available as UPS-1 from Zinc Corporation of America, Monaca, PA |
| 2,5-Dimethyl-2,5-di(t-butylperoxy)-hexane | 50% active, available under the trade designation "VAROX DBPH-50" from R.T.Vanderbilt, Norwalk, CT |
| TAIC | Triallylisocyanurate (98%) available under the trade designation "TAIC" from Nippon Kasei, Japan |
| HFE-7500 | 3-ethoxy-dodecafluoro-2-trifluoromethyl-hexane, available under the trade designation "3M NOVEC ENGINEERED FLUID HFE-7500" from 3M Co., St. Paul, MN, USA. |

### Cure Rheology

Cure rheology tests were carried out using uncured, compounded samples using a rheometer (Alpha Technology RPA 2000 in Moving Die Rheometer (MDR) mode by Alpha Technology, A Dynisco Company, Akron, OH) in accordance with ASTM D 5289-12 at 177°C, no pre-heat, 12 minute elapsed time, and a 0.5 degree arc. Both the minimum torque (ML) and highest torque attained during a specified period of time when no plateau or maximum torque (MH) was obtained were measured. Also measured were the time for the torque to increase 2 units above ML (ts2); the time for the torque to reach a value equal to ML + 0.5(MH - ML), (t'50); and the time for the torque to reach ML + 0.9(MH - ML), (t'90) as well as the tan delta at MH and ML. Results are reported in Table 2.

### Physical Properties

Mooney viscosity was determined by ASTM 1646-00 (ML 1+10@ 121°C). Results are reported in Mooney units.

Press-Cure data are data obtained from mechanical property testing after sheets (150 mm x 150 mm x 2 mm) were pressed and allowed to vulcanize for 10 minutes at 177° C mold temperature. Post-Cure data was obtained from sheets prepared as described for press-cure, which were then further treated by heating the sheets in a circulating air oven maintained at about 230 °C for 4 hours.

Tensile Strength at Break, Elongation at Break, and 100% Modulus (latter is Tensile Strength at 100% Elongation) were determined using a Tensometer 2000 mechanical tester with a 100 kgf load cell in accordance with ASTM D 412-92. The dumbbells for physical properties were cut from the cured sheets with ASTM Die D. All tests were run at a constant cross head displacement rate of 500 mm/min. The values reported were medians of three tests. Durometer or hardness was determined using ASTM D 2240-02 Method A with a Type A-2 Shore Durometer. Units are reported in points.

Compression set resistance was measured on O-rings, according to ASTM D395-03 (method B (25% deformation)) and ASTM D 1414-94. The O-rings were press-cured using a 214 O-ring (AMS AS568) mold at 177° C for 10 minutes and subsequently post cured for 4 hrs at about 230° C. The press-cured and post-cured O-rings were tested for compression set for 22 hours at about 200°C.

### Example 1A: Preparation of 1,3-diiodohexafluoropropane from FSO₂C₃F₆SO₂F

Perfluoropropane disulfonyl fluoride, FSO₂C₃F₆SO₂F, 200g (0.63mol) was reduced in a 3-liter 3-neck round bottom flask equipped with a mechanical stirrer, condenser, addition funnel, and a thermocouple by addition to 97g (2.55mol) sodium borohydride in 690g 2-propanol. The addition rate was done over three hours keeping the reaction temperature below 40°C. After addition, the reaction was heated to 75°C for one hour. The reaction was cooled to 25°C and 375g of 33% sulfuric acid was added followed by filtration to get a HOSOC₃F₆SO₂H solution. A 3-liter 3-neck round bottom flask was charged with iodine, 400g (1.58mol), sodium persulfate 376g, (1.58mol), 600g distilled water, and 200g 2-propanol, stirred and heated to 55°C. The HOSOC₃F₆SO₂H solution then was added over one hour. After addition the reaction was heated to 75°C and held for one hour. Distillation of product and solvent were collected in a receiver by heating the pot mixture up to 108°C. The product and solvent mixture was treated with sodium sulfite, (70g of a 10% aqueous solution) to get to a light yellow solution. Additional water was added to get the fluorochemical to form a lower phase and the fluorochemical product was washed twice with 100g of water. Vacuum distillation gave 1,3-diiodohexafluoropropane, I-C₃F₆-I 165g (0.41mol) having a boiling point of 72°C/100 torr for a 65% yield confirmed by F¹⁹ NMR.

### Example 1B: Preparation of 1,3-diiodohexafluoropropane from HFPO

A 300 mL Hastelloy B-2 autoclave (commercially available from the Superpressure Division of Newport Scientific Inc., Jessup, MD) was charged with 24.5 g of iodine and 2.5 g of nickel catalyst. The autoclave was charged with nitrogen and evacuated three times. The autoclave was cooled down with dry ice and charged with 58 g of HFPO. The autoclave was placed in a rocker where it was heated to 170° C for 12 hrs. The autoclave was allowed to cool to room temperature before the gases produced were vented and 36 g of a dark liquid was obtained. The crude mixture was analyzed by ¹⁹F and ¹H NMR (nuclear magnetic resonance) with the following results: I-CF₂-I (0.0036 absolute wt %), I-(CF₂)₂-I (<0.00005%), I-(CF₂)₃-I (92.2%), I-(CF₂)₄-I (0.41%), I-(CF₂)₅-I (5.4%), I-(CF₂)₈-I (0.27%) plus small amounts of various other monoiodo and diiodo compounds, partially fluorinated compounds, acid fluorides, carboxylic acids, alkenes, etc.

### Example 2A: Preparation of CH₂=CH-C₃F₆-I

A 600ml Parr™ reactor was evacuated and charged with 1,3-diiodohexafluoropropane, I-C₃F₆-I, 100g, (0.25mol made from Example 1A) and t-amyl-2-ethyl hexanoate peroxide 2g, (0.01mol) and stirred. The reactor was heated to 65°C and ethylene 6.9g (0.25mol) was added at 18 psi (pounds per square inch) over one hour and reacted for 20 hrs. The reactor was cooled to 25°C and 85g of product mixture was drained from the reactor. Vacuum distillation gave ICH₂CH₂C₃F₆I, 53g (0.13mol) boiling at 95°C/15torr vacuum. A charge of 50g (0.12mol) of ICH₂CH₂C₃F₆I was reacted in 25g methanol with 28g (0.13mol), 25 % by weight sodium methoxide in methanol, at 65°C for one hour, which gave 1-iodol,1,1,2,2,3,3-hexafluoropentene, IC₃F₆CH=CH₂, 29g (0.1mol) confirmed by ¹⁹F and ¹H NMR, having a boiling point of 106°C.

### Example 2B Preparation of CH₂=CH-C₃F₆-I

A 600ml Parr™ reactor was evacuated and charged with 1,3-diiodohexafluoropropane, I-C₃F₆-I 95g, (0.24mol made from Example 1B) and t-amyl-2-ethyl hexanoate peroxide 3g, (0.01mol) and stirred. The reactor was heated to 65°C and ethylene 6g (0.21mol) was added at 18 psi over one hour and reacted for 20 hrs. The reactor was cooled to 25°C and 93g of product mixture was drained from the reactor. Vacuum distillation isolated ICH₂CH₂C₃F₆I, 51g (0.12mol) boiling at 84°C/6 torr vacuum. A charge of 37g (0.09mol) of ICH₂CH₂C₃F₆I was reacted in 25g methanol with 28g (0.12mol), 25 weight percent sodium methoxide in methanol, at 65°C for one hour, which gave 1-iodol,1,2,2,3,3-hexafluoropentene, IC₃F₆CH=CH₂, 24g, (0.08mol) confirmed by ¹⁹F and ¹H NMR, having a boiling point of 106°C.

### Example 3 Preparation of fluoroelastomer

A 4 liter reactor was charged with 2,250g of water, 2g of ammonium persulfate (APS, (NH₄)₂S₂O₈), 8 g of a 50 wt% aqueous solution of potassium phosphate dibasic (K₂HPO₄) and 3.3 g of oligomeric sulfinate ammonium salt (sulfinate oligomer 1, Example 1 from WO 2012/082707 (Fukushi et al.)). After the reactor was evacuated, the vacuum was broken and the reactor was pressurized with nitrogen to 25 psi (0.17 MPa). This evacuation and pressurization was repeated three times. The reactor was evacuated again and heated to 80°C. The vacuum was broken and the reactor was pressurized to 40 psi (0.28 MPa) with an HFP blend.

The HFP blend consisted of hexaftuoropropylene (HFP); 1,3-diiodohexafluoropropane from Example 1A ; 5-iodohexafluoropentene (CH₂=CH(CF₂)₃I) from Example 2B; and HFE-7500. The HFP blend was prepared by evacuating a 1-liter, stainless steel cylinder and purging it 3 times with nitrogen. After adding 6.7 grams (0.0181 mol) of 1,3-diiodohexafluoropropane, 8.5 grams of 5-iodohexafluoropentene, and 16 grams of HFE-7500 to the reactor, 1,000 grams of HFP was added. This HFP blend was used in the polymer synthesis.

The reactor was then charged with tetrafluoroethylene (TFE), vinylidene fluoride (VDF) and the above described blend of hexafluoropropylene (HFP), bringing reactor pressure to 200 psi (1.38 MPa). Total precharge of TFE, VDF and the HFP blend was 31.6 g, 96.4 g and 229.5 g, respectively. The reactor was agitated at 650 rpm (revolutions per minute). As reactor pressure dropped due to monomer consumption in the polymerization reaction, TFE, VDF, and the HFP blend were continuously fed to the reactor to maintain the pressure at 200 psi (1.38 MPa). The ratios of the HFP blend/ VDF and TFE/ VDF were 0.61 and 0.23 by weight, respectively. After 4.5 hours, the monomers and HFP blend feed were discontinued and the reactor was cooled. The resulting dispersion had a solid content of 31.6 wt. % and a pH of 3.1. The dispersion particle size was 80 nm and total amount of dispersion was 3,878 grams.

For the coagulation, 942 g of the dispersion made as described above was added to 2,320 mL of a 1.25 wt% MgCl₂ in water solution. The crumb was recovered by filtering the coagulate through cheese cloth and gently squeezing to remove excess water. The crumb was rinsed with deionized water and filtered a total of 3 times. After the final rinse and filtration, the crumb was dried in a 130 °C oven for 16 hours. The resulting fluoroelastomer raw gum had a Mooney viscosity of 46.5 at 121°C.

The iodine content by neutron activation analysis was 0.35 wt%. The fluoroelastomer by FT-IR analysis contained 13.2 wt% copolymerized units of TFE, 53.2 wt% copolymerized units of VDF and 33.6 wt% copolymerized units of HFP. The fluorine content was 67.1 wt%.

Mooney viscosity or compound Mooney viscosity was determined in accordance with ASTM D1646-06 TYPE A by a MV 2000 instrument (available from Alpha Technologies, Ohio, USA) using large rotor (ML 1+10) at 121°C. Results are reported in Mooney units.

A fluoroelastomer compound was prepared using a 15.2 cm (6 in) two roll mill by compounding 100 parts of the fluoroelastomer raw gum above with 30 parts of N990 carbon black, 3 parts of zinc oxide, 2 parts of 2,5-dimethyl-2,5-di(t-butylperoxy)-hexane, and 3 parts of TAIC co-agent. During compounding, the polymer was easy to process and the compound did not stick to the roll mill.

The cure rheology of the fluoroelastomer was investigated by testing uncured, compounded mixtures using a mechanical rheometer (available under the trade designation "RPA 2000" from Alpha Technology, A Dynisco Company, Akron, OH) in MDR (Moving Die Rheometer) mode and the procedure described in ASTM D 5289-95. The fluoroelastomer exhibited good curing properties and the 90% cure time (t'90) was 0.9 minutes and delta torque (MH-ML) was 14.9 lb-in (16.8 dNm). The test results are summarized in Table 2.

The fluoroelastomer was press-cured using a 214 O-ring (AMS AS568) mold at 177°C for 5 minutes in air. Then the press-cured O-rings were post cured in air at 230°C for 4 hours. The press-cured and post-cured O-rings were tested for compression set for 22 hours and 70 hours at 200°C in accordance with ASTM D 395-03 Method B and ASTM D 1414-12. The deflection ratio was 25%. The test results are summarized in Table 2.

### Example 4 Preparation of FKM from I-C₄F₈-I and Ex 2B CH₂=CH-C₃F₆-I

A fluoroelastomer gum was prepared and tested as in Example 3 except 7.5 grams (0.0181 mol) of 1,4-diiodooctafluorobutane (I(CF₂)₄I) was used instead of 1,3-diiodohexafluoropropane. The total precharge of TFE, VDF and the HFP blend was 26.5 g 79.7 g and 241.7 g, respectively. The polymerization time was 3.3 hours and the resulting dispersion had a solid content of 30.1 wt. % and a pH of 3.2. The dispersion particle size was 78 nm and the total amount of dispersion was about 3,652 grams. The resulting fluoroelastomer raw gum had a Mooney viscosity of 37.6 at 121°C.

The fluoroelastomer gum was analyzed by FT-IR analysis contained 14.9 wt% of coplymerized units of TFE, 51.5 wt% copolymerized units of VDF and 33.6 wt% copolymerized units of HFP. The fluorine content was 67.4 wt%. The iodine content by neutron activation analysis (NAA) was 0.35wt%. The fluoroelastomer compound in this example exhibited good curing properties and the 90% cure time (t'90) was 1.0 minutes and delta torque (MH-ML) was 15.6 lb-in (17.6 dNm). The test results are summarized in Tables 2.

**Table 2**

| | Example 3 | Example 4 |
|---|---|---|
| Chain transfer agent (CTA) | I(CF₂)₃I | I(CF₂)₄I |
| CTA amount (g) | 6.7 | 7.5 |
| Cure site monomer (CSM) | CH₂=CH(CF₂)₃I | CH₂=CH(CF₂)₃I |
| CSM amount (g) | 8.5 | 8.5 |
| Mooney viscosity [ML 1+10] @121°C | 46.5 | 37.6 |
| Iodine content (wt%) | 0.35 | 0.35 |

| **Cure rheology (MDR) 12 min @177°C** | | |
|---|---|---|
| ML (lb-in) | 1.1 | 0.8 |
| MH (lb-in) | 16.0 | 16.4 |
| Delta torque (lb-in) | 14.9 | 15.6 |
| ts2 (min) | 0.4 | 0.4 |
| t'50 (min) | 0.6 | 0.6 |
| t'90 (min) | 0.9 | 0.9 |
| tandelta ML | 0.9 | 1.0 |
| tandelta MH | 0.125 | 0.112 |

| **Physical properties** | | |
|---|---|---|
| Press cure 10 min. @177°C | | |
| Tensile Strength at break (psi) | 2,044 | 2125 |
| Tensile Strength at break (MPa) | 14.1 | 14.7 |
| Elongation at break (%) | 353 | 335 |
| 100% Modulus (psi) | 501 | 461 |
| 100% Modulus (MPa) | 3.5 | 3.2 |
| Hardness (Shore A) | 69 | 69 |
| Post cure 4 hours @230°C | | |
| Tensile Strength at break (psi) | 3171 | 3114 |
| Tensile Strength at break (MPa) | 21.9 | 21.5 |
| Elongation at break (%) | 275 | 311 |
| 100% Modulus (psi) | 681 | 539 |
| 100% Modulus (MPa) | 4.7 | 3.7 |
| Hardness (Shore A) | 71 | 72 |

| **Compression set** | | |
|---|---|---|
| 22 hours @200°C | | |
| press (%) | 35 | 32 |
| post (%) | 25 | 26 |
| 70 hours @200°C | | |
| press (%) | 45 | 43 |
| post (%) | 36 | 36 |

## Claims

1. A composition comprising a partially fluorinated compound of:
I(CF₂)ₓCH=CH₂ (Formula III)
wherein x is an odd integer selected from 3 to 11.

2. A polymer composition comprising the polymerized reaction product of:
(a) the partially fluorinated compound of claim 1; and
(b) a fluorinated olefinic monomer.

3. The polymer composition of claim 2 , wherein the polymerized reaction product further comprises (c) a chain transfer agent, wherein the chain transfer agent is selected from the group consisting of: a C1 to C10 α,ω-diiodoperfluoroalkane; I(CF₂)_{y}CH₂CH₂I, wherein y is an integer from 2 to 10; CH₂I₂; and combinations thereof.

4. The polymer composition of claim 3, wherein the chain transfer agent is 1,3-diiodoperfluoropropane or 1,4-diiodoperfluorobutane.

5. The polymer composition of any one of claims 2 to 4, wherein x is 3.

6. The polymer composition of any one of claims 2 to 5, wherein the polymer composition comprises 0.05 to 1 % by weight of iodine.

7. An article comprising the cured polymer composition according to any one of claims 2 to 6.

8. A method of making a polymer comprising:
(a) providing a partially fluorinated compound, I(CF₂)ₓCH=CH₂ (Formula III) wherein x is an odd integer selected from 3 to 11; a fluorinated olefinic monomer; and an initiator; and
(b) polymerizing the partially fluorinated compound and the fluorinated olefinic monomer to form a polymer.

9. The method of claim 8, further comprising polymerizing in the presence of a chain transfer agent.

10. The method of claim 9, wherein the chain transfer agent is 1,3-diiodoperfluoropropane or 1,4-diiodoperfluorobutane.

11. The method of any one of claims 8 to 10, wherein the fluorinated olefinic monomer is selected from the group consisting of: hexafluoropropylene, trifluoroethylene, fluoroethylene, vinylidene fluoride, tetrafluoroethylene, perfluoro(methyl vinyl ether), perfluoro(propyl vinyl ether), perfluoro(methoxypropyl vinyl ether), perfluoro(ethoxymethyl vinyl ether), chlorotrifluoroethylene, and combinations thereof.

12. A method of making a partially fluorinated compound comprising reacting a molecule of the formula I(CF₂)ₓI with ethylene to form I(CF₂)ₓCH₂CH₂I, wherein x is an odd integer selected from 3 to 11, and dehydroiodinating I(CF₂)ₓCH₂CH₂I to form an partially fluorinated iodo alkene compound of the formula I(CF₂)ₓCH=CH₂ wherein x is an odd integer selected from 3 to 11.

## Patentansprüche

1. Zusammensetzung, umfassend eine teilfluorierte Verbindung von:
I(CF₂)ₓCH=CH₂ (Formel III),
wobei x eine ungerade ganze Zahl ist, ausgewählt aus 3 bis 11.

2. Polymerzusammensetzung, umfassend das polymerisierte Reaktionsprodukt von:
(a) der teilfluorierten Verbindung nach Anspruch 1; und
(b) einem fluorierten Olefinmonomer.

3. Polymerzusammensetzung nach Anspruch 2, wobei das polymerisierte Reaktionsprodukt ferner umfasst:
(c) ein Kettenübertragungsmittel, wobei das Kettenübertragungsmittel ausgewählt ist aus der Gruppe, bestehend aus: einem C1 bis C10-α,ω-Diiodperfluoralkan; I(CF₂)_{y}CH₂CH₂I, wobei y eine ganze Zahl von 2 bis 10 ist; CH₂I₂; und Kombinationen davon.

4. Polymerzusammensetzung nach Anspruch 3, wobei das Kettenübertragungsmittel 1,3-Diiodperfluorpropan oder 1,4-Diiodperfluorbutan ist.

5. Polymerzusammensetzung nach einem der Ansprüche 2 bis 4, wobei x 3 ist.

6. Polymerzusammensetzung nach einem der Ansprüche 2 bis 5, wobei die Polymerzusammensetzung 0,05 bis 1 Gew.-% Iod umfasst.

7. Gegenstand, umfassend die gehärtete Polymerzusammensetzung nach einem der Ansprüche 2 bis 6.

8. Verfahren zur Herstellung eines Polymers, umfassend:
(a) Bereitstellen einer teilfluorierten Verbindung, I(CF₂)ₓCH=CH₂ (Formel III) wobei x ein ungerade ganze Zahl ist, ausgewählt aus 3 bis 11; eines fluorierten Olefinmonomers; und eines Starters; und
(b) Polymerisieren der teilfluorierten Verbindung und des fluorierten Olefinmonomers zur Bildung eines Polymers.

9. Verfahren nach Anspruch 8, weiterhin umfassend das Polymerisieren in Gegenwart eines Kettenübertragungsmittels.

10. Verfahren nach Anspruch 9, wobei das Kettenübertragungsmittel 1,3-Diiodoperfluorpropan oder 1,4-Diiodoperfluorbutan ist.

11. Verfahren nach einem der Ansprüche 8 bis 10, wobei das fluorierte Olefinmonomer ausgewählt ist aus der Gruppe, bestehend aus: Hexafluorpropylen, Trifluorethylen, Fluorethylen, Vinylidenfluorid, Tetrafluorethylen, Perfluor(methylvinylether), Perfluor(propylvinylether), Perfluor(methoxypropylvinylether), Perfluor(ethoxymethylvinylether), Chlortrifluorethylen, und Kombinationen davon.

12. Verfahren zur Herstellung einer teilfluorierten Verbindung, umfassend die Umsetzung eines Moleküls der Formel I(CF₂)ₓI mit Ethylen zur Bildung von I(CF₂)ₓCH₂CH₂I, wobei x eine ungerade ganze Zahl ist, ausgewählt aus 3 bis 11, und die Dehydroiodierung von I(CF₂)ₓCH₂CH₂I zur Bildung einer teilfluorierten Iodalken-Verbindung der Formel I(CF₂)ₓCH=CH₂, wobei x eine ungerade ganze Zahl ist, die aus 3 bis 11 ausgewählt ist.

## Revendications

1. Composition comprenant un composé partiellement fluoré de :
I(CF₂)ₓCH=CH₂ (Formule III)
dans laquelle x est un nombre entier impair choisi de 3 à 11.

2. Composition polymère comprenant le produit de réaction polymérisé :
(a) du composé partiellement fluoré selon la revendication 1 ; et
(b) d'un monomère oléfinique fluoré.

3. Composition polymère selon la revendication 2, dans laquelle le produit de réaction polymérisé comprend en outre (c) un agent de transfert de chaîne, dans laquelle l'agent de transfert de chaîne est choisi dans le groupe constitué de : un α,ω-di-iodoperfluoroalcane en C1 à C10 ; I(CF₂)_{y}CH₂CH₂I, dans laquelle y est un nombre entier allant de 2 à 10 ; CH₂I₂ ; et des combinaisons de ceux-ci.

4. Composition polymère selon la revendication 3, dans laquelle l'agent de transfert de chaîne est le 1,3-di-iodoperfluoropropane ou le 1,4-di-iodoperfluorobutane.

5. Composition polymère selon l'une quelconque des revendications 2 à 4, dans laquelle x vaut 3.

6. Composition polymère selon l'une quelconque des revendications 2 à 5, où la composition polymère comprend 0,05 à 1 % en poids d'iode.

7. Article comprenant la composition polymère durcie selon l'une quelconque des revendications 2 à 6.

8. Procédé de fabrication d'un polymère, comprenant :
(a) la fourniture d'un composé partiellement fluoré, I(CF₂)ₓCH=CH₂ (Formule III) où x est un nombre entier impair choisi de 3 à 11 ; un monomère oléfinique fluoré ; et un inducteur ; et
(b) la polymérisation du composé partiellement fluoré et du monomère oléfinique fluoré pour former un polymère.

9. Procédé selon la revendication 8, comprenant en outre la polymérisation en présence d'un agent de transfert de chaîne.

10. Procédé selon la revendication 9, dans lequel l'agent de transfert de chaîne est le 1,3-di-iodoperfluoropropane ou le 1,4-di-iodoperfluorobutane.

11. Procédé selon l'une quelconque des revendications 8 à 10, dans lequel le monomère oléfinique fluoré est choisi dans le groupe constitué de : hexafluoropropylène, trifluoroéthylène, fluoréthylène, fluorure de vinylidène, tétrafluoroéthylène, perfluoro(méthyl-vinyl-éther), perfluoro(propyl-vinyl-éther), perfluoro(méthoxypropyl-vinyl-éther), perfluoro(éthoxyméthyl-vinyl-éther), chlorotrifluoréthylène et des combinaisons de ceux-ci.

12. Procédé de fabrication d'un composé partiellement fluoré comprenant la réaction d'une molécule de formule I(CF₂)ₓI avec de l'éthylène pour former I(CF₂)ₓCH₂CH₂I, dans lequel x est un nombre entier impair choisi de 3 à 11, et la déshydro-iodation de I(CF₂)ₓCH₂CH₂I pour former un composé iodoalcène partiellement fluoré de formule I(CF₂)ₓCH=CH₂ dans laquelle x est un nombre entier impair choisi de 3 à 11.
